## Europäisches Patentamt
## European Patent Office
## Office européen des brevets

(11) Numéro de publication: **0 077 223**
**A2**

(12)
# DEMANDE DE BREVET EUROPEEN

(21) Numéro de dépôt: 82401434.4

(22) Date de dépôt: 30.07.82

(51) Int. Cl.³: **C 03 C 7/00, A 61 L 17/00**

(30) Priorité: 14.08.81 FR 8115726
21.04.82 FR 8206818

(43) Date de publication de la demande: 20.04.83
Bulletin 83/16

(84) Etats contractants désignés: BE CH DE GB LI NL

(71) Demandeur: BOUSSOIS S.A., 126-130 rue Jules Guesde,
F-92302 Levallois-Perret (FR)
Demandeur: Simonpaoli, Yves, 5, Place du 18 Juin,
F-75006 Paris (FR)

(72) Inventeur: Hochart, Paul, 3, Résidence de l'Orangerie,
F-78170 La Celle Saint-Cloud (FR)
Inventeur: Simonpaoli, Yves, 5, Place du 18 Juin,
F-75006 Paris (FR)

(74) Mandataire: Bouju, André, 38 Avenue de la Grande
Armée, F-75017 Paris (FR)

(54) Prothèse métallique revêtue par un émail vitreux et procédé pour la réaliser.

(57) La prothèse métallique revêtue par un émail vitreux est réalisée en un alliage à base de nickel et de chrome, tel que la somme des concentrations de nickel et de chrome soit supérieure ou égale à 80% et que le rapport de la concentration de nickel sur celle de chrome soit supérieure ou égale à 2,5, et la composition de l'émail vitreux est la suivante:

$SiO_2$: 44 à 54%
$B_2O_3$: 16 à 30%
$Na_2O$: 13 à 20%
$K_2O$: 0 à 4%
$Li_2O$: 0 à 2,5%
$CaO$: 0 à 3%
$F_2Ca$: 0 à 5%
$Al_2O_3$: 3 à 9%
$ZrO_2$: 0 à 6%
$NiO$: 0 à 2%
$CoO$: 0 à 1,5%
$MnO_2$: 0 à 1,5%

Utilisation pour les prothèses dentaires.

Prothèse métallique revêtue par un
émail vitreux et procédé pour la réaliser

La présente invention concerne une prothèse
dentaire métallique, revêtue par un émail vitreux.

L'invention vise également un procédé pour
réaliser une telle prothèse.

Les prothèses métalliques, revêtues d'émail,
connues jusqu'à présent, sont réalisées en or ou en
alliage d'or et d'autres métaux précieux.

L'émaillage de ces prothèses est réalisé par
cuisson, généralement vers 900-980°C d'une céramique
ou d'un émail obtenu à partir d'une composition de
verre boro-silicaté et d'autres oxydes.

L'or et les alliages d'or et de métaux précieux utilisés pour ces prothèses présentent deux inconvénients majeurs. D'une part, leur module d'élasticité
est incompatible avec la réalisation de prothèses ou
"bridges" de longue portée et d'autre part, leur prix
est devenu très élevé.

C'est ainsi que depuis une dizaine d'années
on a cherché à remplacer l'or et ses alliages par des
alliages inoxydables, moins coûteux et présentant des
propriétés mécaniques supérieures.

A cet égard, les alliages riches en nickel et
chrome présentent les qualités requises. Cependant les
émaux dentaires utilisés jusqu'à présent pour revêtir
les prothèses en or ou en alliage d'or n'adhèrent pas
aux alliages précités à base de nickel et de chrome.

Il a été possible d'obtenir une certaine
adhérence de l'émail sur de tels alliages en pratiquant sur la surface de ces derniers des aspérités, telles que des stries. Toutefois, l'adhérence obtenue à l'aide de tels artifices est loin d'atteindre celle obtenue lorsque le support de la prothèse est en or ou en alliage d'or.

Le but de la présente invention est de créer une composition d'émail vitreux permettant d'obtenir une excellente adhérence sur des alliages relativement peu onéreux et présentant de bonnes propriétés mécaniques telles que les alliages riches en nickel et chrome.

Suivant l'invention, la prothèse métallique revêtue par un émail vitreux est caractérisée en ce qu'elle est réalisée en un alliage à base de nickel et de chrome, tel que la somme des concentrations de nickel et de chrome soit supérieure ou égale à 80% et que le rapport de la concentration de nickel sur celle de chrome soit supérieure ou égale à 2,5, et en ce que la composition de l'émail vitreux est la suivante:

| | |
|---|---|
| $SiO_2$: | 44 à 54% |
| $B_2O_3$: | 16 à 30% |
| $Na_2O$: | 13 à 20% |
| $K_2O$ : | 0 à 4% |
| $Li_2O$: | 0 à 2,5% |
| CaO : | 0 à 3% |
| $F_2Ca$: | 0 à 5% |
| $Al_2O_3$: | 3 à 9% |
| $ZrO_2$: | 0 à 6% |
| NiO : | 0 à 2% |
| CoO : | 0 à 1,5% |
| $MnO_2$: | 0 à 1,5% |

Une telle composition d'émail présente une excellente adhérence, en particulier sur des supports en alliage à base de nickel et de chrome répondant aux concentrations précitées.

Cette remarquable adhérence peut s'expliquer d'une part, par la température de cuisson relativement basse de l'émail qui permet de limiter l'oxydation superficielle de l'alliage et d'obtenir une liaison chimique stable avec ce dernier, grâce à la labilité des ions alcalins contenus dans l'émail.

La présence d'oxydes alcalins ($Na_2O$ et $K_2O$) permet à la fois d'abaisser la température de cuisson de l'émail et d'obtenir pour celui-ci un coefficient de dilatation thermique compris entre 95 et $110.10^{-7}$ (unités CGS), qui est compatible avec l'alliage Ni Cr de la prothèse, ce qui permet de limiter les contraintes susceptibles d'être engendrées entre la couche d'émail et l'alliage, sous l'effet des variations de température.

Selon une version préférée de l'invention, l'alliage de la prothèse présente la composition suivante:

| | |
|---|---|
| Cr: | 12 à 40% |
| Ni: | 50 à 80% |
| C : | 0,05 à 0,2% |
| Si: | 0,05 à 0,5% |
| Mo: | 0 à 0,1% |
| Fe: | 0 à 15% |
| B : | 0 à 3% |
| Ti: | 0 à 5% |
| Co: | 0 à 10% |
| Zr: | 0 à 5% |
| Nb: | 0 à 5% |

Un tel alliage présente de très bonnes propriétés mécaniques et présente une excellente adhérence avec l'émail obtenu à partir de la composition conforme à l'invention.

Selon l'une des versions du procédé conforme à l'invention, on applique sur l'alliage à base de nickel et de chrome une première couche d'un émail obtenu à partir d'une composition conforme à l'invention, additionnée d'agents opacifiants et/ou dévitrifiants tels

que $SnO_2$ et $ZrO_2$, puis on applique une seconde couche d'émail dont le verre de base peut être différent suivant les dents à réaliser.

On utilise le verre, constituant principal de la première couche d'émail mais avec moins de charges minérales pour lui conserver une certaine translucidité. On lui ajoute, à faible dose, des agents colorants tels que NiO ou $TiO_2$ pour lui conférer la coloration souhaitée.

On utilise un verre de composition différente du précédent quand il s'agit de réaliser une dent telle l'incisive, dont la forme et la translucidité sont nettement déterminées. Comme précédemment, on y ajoute une faible dose d'agents opacifiants et de colorants.

La composition d'émail précitée convient bien en tant que première couche grâce à son excellente adhérence. Toutefois, cette couche n'est pas suffisamment translucide et n'est pas assez résistante vis-à-vis des agents chimiques.

De préférence cette première couche est recouverte par une seconde couche d'émail renfermant les composés principaux suivants:

| | |
|---|---|
| $SiO_2$: | 45 à 68% |
| $B_2O_3$: | 0 à 8% |
| $Na_2O$: | 10 à 17% |
| $K_2O$ : | 10 à 20% |

Cette composition d'émail diffère de celle de la seconde couche d'émail décrite précédemment, principalement par une teneur nettement plus élevée en $K_2O$ (oxyde de potassium).

On a constaté de façon surprenante que cette composition d'émail permettait d'obtenir après cuisson, une seconde couche parfaitement translucide, adhérant bien à la première couche d'émail et présentant une excellente résistance aux agents chimiques.

La composition précitée ne convient pas pour réaliser la première couche d'émail. En effet, les essais ont montré qu'une telle première couche n'adhère pas à la couche superficielle oxydée de l'alliage en nickel et Chrome de la prothèse. Ce résultat peut s'expliquer par le fait que les atomes de potassium en raison de leur moins grande mobilité que les atomes de sodium, ne permettent pas, contrairement à ceux de sodium, de réaliser un échange par diffusion réciproque avec les oxydes métalliques de la couche oxydée précitée.

Par contre, la seconde couche obtenue à partir de la composition précitée présente une adhérence exceptionnelle sur la première couche d'émail.

D'autres particularités et avantages de l'invention apparaîtront encore dans la description ci-après.

On donne ci-après quelques exemples non limitatifs de réalisation de l'invention.

EXEMPLE 1

Prothèse émaillée à base d'alliage de nickel et de chrome.

La composition de cet alliage est la suivante:

| | |
|---|---|
| Cr: | 21% |
| Ni: | 62% |
| C : | 0,05% |
| Fe: | 11% |
| B : | 3% |
| Ti: | 3% |
| Mn: | 1% |

Cet alliage possède des propriétés mécaniques compatibles avec la réalisation de prothèses ou "bridges" de longue portée.

Pour émailler cet alliage, on part d'un verre borosilicaté de composition suivante:

| | |
|---|---|
| $SiO_2$: | 52% |
| $Na_2O$: | 18% |
| $K_2O$ : | 1,5% |
| $Li_2O$: | 2,5% |
| $Al_2O_3$: | 4% |
| $B_2O_3$: | 18% |
| CaO : | 2% |
| $F_2Ca$: | 2% |

On applique sur l'alliage une première couche d'émail, d'épaisseur inférieure ou égale à 0,5 mm environ, obtenu par cuisson à 780°C environ du mélange suivant:

| | |
|---|---|
| Verre précité: | 70% |
| $SnO_2$: | 12% |
| $ZrO_2$: | 12% |
| $Al_2O_3$: | 8% |

L'oxyde d'étain ($SnO_2$) ajouté à la composition joue le rôle d'opacifiant. L'oxyde de zirconium ($ZrO_2$) joue à la fois le rôle d'opacifiant et d'agent de dévitrification.

On applique une seconde et éventuellement une troisième couche d'émail, obtenu par cuisson à 795°C sous un vide partiel de 10 mbar du mélange suivant:

| | |
|---|---|
| Verre précité: | 89% |
| $TiO_2$: | 1% |
| $SiO_2$: | 10% |
| NiO : | 0,004% |

L'oxyde de nickel (NiO) et l'oxyde de titane ($TiO_2$) ajoutés à la composition confèrent à l'émail la couleur voulue.

L'émail appliqué sur la prothèse présente un coefficient de dilatation thermique voisin de $100.10^{-7}$ (unités CGS).

Cet émail présente une excellente adhérence. Cette dernière est démontrée en pliant à angle droit une lame d'alliage à base de nickel et de chrome revêtue par l'émail. Ce dernier se fissure dans la zone du pli, en formant des blocs d'émail disjoints qui, pour la plupart restent adhérents au support.

Les essais ont montré d'autre part que l'adhérence de l'émail au support était toujours excellente lorsque les proportions des constituants de la composition variaient entre les limites indiquées ci-après:

| | |
|---|---|
| $SiO_2$ : | 44 à 54% |
| $B_2O_3$ : | 16 à 30% |
| $Na_2O$ : | 13 à 20% |
| $K_2O$ : | 0 à 4% |
| $Li_2O$ : | 0 à 2,5% |
| $CaO$ : | 0 à 3% |
| $F_2Ca$ : | 0 à 5% |
| $Al_2O_3$ : | 3 à 9% |
| $ZrO_2$ : | 0 à 6% |
| $NiO$ : | 0 à 2% |
| $CoO$ : | 0 à 1,5% |
| $MnO_2$ : | 0 à 1,5% |

L'émail obtenu à partir d'une telle composition présente une température de cuisson inférieure à 800°C et un coefficient de dilatation thermique compris entre 95 et $110.10^{-7}$ (unités CGS).

Par ailleurs, l'adhérence d'un tel émail reste tout aussi excellente lorsque les constituants de l'alliage à base de nickel et de chrome varient entre les limites ci-après:

| | |
|---|---|
| $Cr$ : | 12 à 40% |
| $Ni$ : | 50 à 80% |
| $C$ : | 0,05 à 0,2% |
| $Si$ : | 0,05 à 0,5% |

8         0077223

| | |
|---|---|
| Mo: | 0 à 0,1% |
| Fe: | 0 à 15% |
| B : | 0 à 3% |
| Ti: | 0 à 5% |
| Co: | 0 à 10% |
| Zr: | 0 à 5% |
| Nb: | 0 à 5% |
| Mn: | 0 à 1% |
| Al: | 0 à 3% |

EXEMPLE 2

On procède comme dans l'exemple 1, en appliquant sur la première couche d'émail, une seconde couche réalisée à partir de la composition suivante:

| | |
|---|---|
| $SiO_2$: | 52,3% |
| $Na_2O$: | 13,7% |
| $K_2O$: | 17,5% |
| $CaF_2$: | 2,8% |
| BaO: | 4,4% |
| $ZrO_2$: | 10,0% |

L'émail obtenu par cuisson à une température comprise entre 740 et 760°C de la composition précitée présente un coefficient de dilatation thermique compris entre 120 et 125 x $10^{-7}$ (unités CGS).

On peut ajouter à la composition précitée, avant la cuisson de cette dernière, les ingrédients suivants:

| | |
|---|---|
| $TiO_2$: | 0,8% |
| NiO: | 0,005% |
| $SiO_2$: | 7,2% |
| $Fe_2O_3$: | 0,002% |

Ces ingrédients permettent de donner à l'émail une coloration légèrement gris-jaunâtre.

L'épaisseur de cette seconde couche d'émail peut varier, comme pour la première couche, entre 0,5 et 3 mm suivant la dimension et la nature des dents.

0077223

REVENDICATIONS

1. Prothèse métallique revêtue par un émail vitreux, caractérisée en ce qu'elle est réalisée en un alliage à base de nickel et de chrome, tel que la somme des concentrations de nickel et de chrome soit supérieure ou égale à 80% et que le rapport de la concentration de nickel sur celle de chrome soit supérieure ou égale à 2,5, et en ce que la composition de l'émail vitreux est la suivante:

| | |
|---|---|
| $SiO_2$: | 44 à 54% |
| $B_2O_3$: | 16 à 30% |
| $Na_2O$: | 13 à 20% |
| $K_2O$ : | 0 à 4% |
| $Li_2O$: | 0 à 2,5% |
| CaO : | 0 à 3% |
| $F_2Ca$: | 0 à 5% |
| $Al_2O_3$: | 3 à 9% |
| $ZrO_2$: | 0 à 6% |
| NiO : | 0 à 2% |
| CoO : | 0 à 1,5% |
| $MnO_2$: | 0 à 1,5% |

2. Prothèse conforme à la revendication 1, caractérisée en ce que l'alliage présente la composition suivante:

| | |
|---|---|
| Cr: | 12 à 40% |
| Ni: | 50 à 80% |
| C : | 0,05 à 0,2% |
| Si: | 0,05 à 0,5% |
| Mo: | 0 à 0,1% |
| Fe: | 0 à 15% |
| B : | 0 à 3% |
| Ti: | 0 à 5% |
| Co: | 0 à 10% |
| Zr: | 0 à 5% |
| Nb: | 0 à 5% |

Mn:                     0 à  1%

Al:                     0 à  3%

3. Procédé pour réaliser une prothèse émaillée, conforme à l'une quelconque des revendications 1 ou 2, caractérisé en ce qu'on applique sur l'alliage de base de la prothèse une première couche d'un émail obtenu à partir de ladite composition additionnée d'agent opacifiants et/ou dévitrifiants tels que $SnO_2$ et $ZrO_2$ et on applique ensuite une seconde couche d'un émail obtenu à partir de la composition précitée additionnée d'agents tels que NiO ou $TiO_2$ apportant à l'émail la coloration voulue.

4. Procédé pour réaliser une prothèse émaillée, conforme à l'une quelconque des revendications 1 ou 2, caractérisé en ce qu'on applique sur l'alliage de base de la prothèse une première couche d'un émail obtenu à partir de ladite composition additionnée d'agents opacifiants et/ou dévitrifiants tels que $SnO_2$ et $ZrO_2$ et on applique ensuite une seconde couche d'un émail obtenu à partir d'un verre répondant sensiblement à la composition suivante:

SiO_2:                    64%

Na_2O:                    14%

K_2O:                      4%

CaO:                      1,8%

CaF_2:                    1,2%

BaO:                      5,2%

ZrO_2:                    9,8%

ce verre étant éventuellement chargé d'agents opacifiants et/ou colorants.

5. Composition d'émail vitreux, destinée à recouvrir une première couche d'émail conforme à la revendication 1, caractérisée en ce que la composition de la seconde couche renferme les composés suivants:

| | |
|---|---|
| $SiO_2$: | 45 à 68% |
| $B_2O_3$: | 0 à 8% |
| $Na_2O$: | 10 à 17% |
| $K_2O$: | 10 à 20% |

6. Composition conforme à la revendication 5, caractérisée en ce qu'elle renferme les composés suivants:

| | |
|---|---|
| $SiO_2$: | 52,3% |
| $Na_2O$: | 13,7% |
| $K_2O$: | 17,5% |
| $CaF_2$: | 2,8% |
| $BaO$: | 4,4% |
| $ZrO_2$: | 10% |